# EUROPÄISCHE PATENTSCHRIFT

(11) **EP 1 070 717 B1**
(45) Veröffentlichungstag und Bekanntmachung des Hinweises auf die Patenterteilung: **27.02.2002**
(21) Anmeldenummer: 00114475.7
(22) Anmeldetag: 06.07.2000
(51) Int. Cl.: C07D 487/08

(54) **Verfahren zur Herstellung von reinem Triethylendiamin**
Process for the preparation of pure triethylenediamine
Procédé de préparation de triéthylènediamine pure

(30) Priorität: 23.07.1999 DE 19933850; 22.12.1999 DE 19962455
(43) Veröffentlichungstag der Anmeldung: 24.01.2001
(73) Patentinhaber: BASF AKTIENGESELLSCHAFT, 67056 Ludwigshafen (DE)
(72) Erfinder: Riechers, Hartmut, Dr., 67435 Neustadt (DE); Höhn, Arthur, Dr., 67281 Kirchheim (DE); Simon, Joachim, Dr., 68161 Mannheim (DE); Lang, Ortmund, 66903 Ohmbach (DE); Schoenmakers, Hartmut, Dr., 69121 Heidelberg (DE); Rauls, Matthias, Dr., 67117 Limburgerhof (DE); Claerbout, Koen, 9130 Verrebroek (BE)

(56) Entgegenhaltungen:
- EP-A- 0 111 928
- US-A- 4 233 447
- US-A- 5 741 906

## Beschreibung

Die vorliegende Erfindung betrifft ein Verfahren zur Herstellung von reinem Triethylendiamin (= TEDA = DABCO® = 1,4-Diazabicyclo-[2,2,2]-octan) und Lösungen hiervon.

Triethylendiamin (TEDA), das unter Normalbedingungen ein Feststoff ist, ist ein bedeutender Katalysator für die Herstellung von Polyurethanschäumen.

Für diese und andere Einsatzgebiete ist ein reines, möglichst geruchloses und reinweißes TEDA mit einer möglichst geringen Verfärbung, z. B. mit einer möglichst kleinen APHA-Farbzahl (DIN-ISO 6271), das diese Eigenschaften auch über längere Lagerzeiten (von z. B. 6, 12 oder mehr Monaten) beibehält, erwünscht.

Zur Herstellung und Reinigung von TEDA sind verschiedene Verfahren bekannt:

DT-A-24 42 929 betrifft ein Verfahren zur Herstellung von TEDA durch Abspaltung von Glykol aus N,N'-Di(hydroxyethyl)piperazin in Gegenwart von Al₂O₃ als Katalysator.

US-A-3,297,701 offenbart ein Verfahren zur Herstellung von Diazabicyclo-[2,2,2]-octanen durch Umsetzung entsprechender Hydroxyethyl- oder Aminoethyl-piperazine bei erhöhter Temperatur in Gegenwart von Metallphosphaten, wie z. B. Calciumphosphat.

DE-A-36 34 258 beschreibt ein Verfahren zur Herstellung von Diazabicyclo-[2,2,2]-octanen durch Umsetzung entsprechender Hydroxyethyl- oder Aminoethyl-piperazine in Gegenwart von Zirkoniumphosphaten.

DE-A-1 745 627 betrifft ein Verfahren zur Herstellung von TEDA und Piperazin durch Umsetzung eines Ethylendiamins an einem sauren Kieselsäure-Tonerde-Katalysator bei erhöhter Temperatur und Gewinnung des TEDAs durch Destillation und/oder Kristallisation.

DE-A-37 18 395 beschreibt die Herstellung von TEDA durch Umsetzung eines acyclischen Hydroxyethyl-ethylenpolyamins und/oder cyclischen Hydroxyethyl-ethylenpolyamins in Gegenwart eines phosphorhaltigen Titandioxid- oder Zirkoniumdioxid-Katalysators.

EP-A-111 928 beschreibt die Verwendung von bestimmten Phosphatkatalysatoren, wie z. B. Mono- oder Pyrophosphate des Magnesiums, Calciums, Bariums oder Aluminiums, bei organischen Kondensationsreaktionen, wie z. B. der Umsetzung von N-(2-Hydroxyethyl)-piperazin zu TEDA.

EP-A-382 055 offenbart ein Verfahren zur Herstellung von TEDA, wobei man 1,2-Diaminoethan und 0 bis 200 Mol-% Piperazin an Al-, B-, Ga- und/oder Fe-silikatzeolithen bei erhöhter Temperatur umsetzt.

EP-A-842 935 beschreibt ein Verfahren zur Herstellung von TEDA durch Umsetzung einer Aminverbindung, wie Monoethanolamin, an einem Katalysator zu einem Produkt enthaltend TEDA und Piperazin und anschließende Umsetzung dieses Produkts mit einer ethylierenden Verbindung, die zumindest ein N und/oder O-Atom enthält, in Gegenwart eines formselektiven Zeolithkatalysators.

US-A-5,741,906 betrifft die Herstellung von TEDA durch Umsetzung einer Aminverbindung, wie Monoethanolamin, an einem Zeolithkatalysator vom Pentasil-Typ.

Die bekannten Verfahren zur TEDA-Herstellung führen zur Bildung roher Umsetzungsprodukte, die neben TEDA noch Wasser, Nebenprodukte, wie z. B. Piperazin und hochmolekulare Polymerisate, sowie ein gegebenenfalls bei der Umsetzung eingesetztes Lösungsmittel enthalten. TEDA wird aus diesen Gemischen gewöhnlich durch diskontinuierliche oder kontinuierliche Destillation oder Rektifikation abgetrennt und meist in einem anschließenden Schritt durch Kristallisation oder Umkristallisation gereinigt.

TEDA ist aufgrund seiner Eigenschaften [hygroskopisch, temperaturempfindlich, Siedepunkt (174 °C bei Normaldruck) und Schmelzpunkt (158-160 °C) liegen dicht beieinander] schwierig und nur unter entsprechendem technischem Aufwand zu handhaben, ohne dass eine Verschlechterung der Qualität des TEDAs bezüglich Farbe, Farbstabilität (unerwünschte Zunahme der Farbzahl, z. B. gemessen als APHA-Farbzahl, über die Lagerzeit), Geruch (unerwünschter Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen) und Reinheit auftritt.

Das nach den bekannten Verfahren nach einer Destillation oder Rektifikation erhaltene TEDA und hieraus hergestellte Lösungen ist/sind aufgrund der Farbe (z. B. gemessen als APHA-Farbzahl), Farbstabilität und/oder des Geruches meist nicht marktfähig und nur eine weitere Reinigungsstufe, wie eine technisch aufwendige Kristallisation oder Umkristallisation, kann die TEDA-Qualität verbessern.

Es hat daher nicht an Versuchen gefehlt, alternative Verfahren aufzufinden, die TEDA in verbesserter Qualität bereitstellen.

DT-A-26 11 069 betrifft die Gewinnung von TEDA, wobei man dem rohen TEDA Propylenglykol zusetzt und anschließend fraktionierend destilliert.

DE-A-28 49 993 offenbart ein Verfahren zur Abtrennung und Gewinnung von TEDA, wobei man dem rohen TEDA Wasser zusetzt und anschließend destilliert.

JP-A-49 048 609 beansprucht ein Verfahren zur Reinigung von Piperazin und/oder TEDA durch fraktionierte Destillation einer Mischung enthaltend Piperazin und/oder TEDA, umfassend die Schritte Auflösen der Piperazin- und/oder TEDA-Destillate in Wasser oder einem organischen Lösungsmittel, wobei das Lösungsmittel flüssig oder gasförmig vorliegen kann, und Sammeln der Lösungen der Destillate. Mit diesem Verfahren soll anmeldungsgemäß die Aufgabe gelöst werden, Verstopfungen durch Feststoffe in der Destillationsapparatur zu verhindern. Die Beschreibung, die schematischen Darstellung der Destillationsapparatur und die Beispiele in dieser Patentanmeldung lehren, dass hierzu das Piperazin oder das TEDA am Kopf der Destillationskolonne in einem Kondensator zunächst verflüssigt und erst danach in dem Lösungsmittel aufgelöst wird.

Nachteilig an diesen Verfahren ist, dass sie das TEDA nicht in der erwünschten Qualität liefern.

Der vorliegenden Erfindung lag die Aufgabe zugrunde, ein verbessertes, effizientes und wirtschaftliches Verfahren zur Herstellung von reinem Triethylendiamin (TEDA) und Lösungen hiervon aufzufinden, das TEDA und TEDA-Lösungen mit verbesserter Qualität bezüglich Farbe, Farbstabilität, Geruch und Reinheit liefert.

Demgemäß wurde ein Verfahren zur Herstellung einer Lösung von reinem Triethylendiamin (TEDA) gefunden, welches dadurch gekennzeichnet ist, dass man TEDA verdampft und das dampfförmige TEDA in ein flüssiges Lösungsmittel einleitet. Durch anschließende Auskristallisation des TEDAs aus der so erhaltenen Lösung wird reines TEDA mit der aufgabengemäß verbesserten Qualität erhalten.

Durch das erfindungsgemäße Verfahren, wobei die Einleitung des dampfförmigen TEDAs in ein flüssiges Lösungsmittel im Folgenden auch TEDA-Quench genannt wird, wird die Bildung von unerwünschten Nebenprodukten, die zur Qualitätsminderung führen, entscheidend verringert. Der flüssige Aggregatzustand des TEDAs am Ausgang der Verdampfungs- oder Destillationsapparatur wird erfindungsgemäß vermieden, die bei Destillationen übliche Verflüssigung des Destillats findet nicht statt.

Als Lösungsmittel für diesen TEDA-Quench eignen sich besonders cyclische oder acyclische (= aliphatische) Kohlenwasserstoffe (insbesondere verzweigte oder unverzweigte Alkane oder Alkangemische, wie z. B. n-Pentan, iso-Pentan, Cyclopentan, Hexan, Cyclohexan, Heptan, Octan, Petrolether), chlorierte aliphatische Kohlenwasserstoffe (insbesondere chlorierte Alkane, wie z. B. Dichlormethan, Trichlormethan, Dichlorethan, Trichlorethan), aromatische Kohlenwasserstoffe (wie z. B. Benzol, Toluol, Xylole), chlorierte aromatische Kohlenwasserstoffe (wie z. B. Chlorbenzol), Alkohole (wie z. B. Methanol, Ethanol, Ethylenglykol, 1,4-Butandiol, und Polyetheralkohole, insbesondere Polyalkylenglykole, wie Diethylenglykol, Dipropylenglykol), Ketone (insbesondere aliphatische Ketone, wie Aceton, Methylethylketon, Diethylketon), aliphatische Carbonsäureester (wie z. B. Essigsäuremethylester, Essigsäureethylester), aliphatische Nitrile (wie z. B. Acetonitril, Propionitril), Ether (wie z. B. Dioxan, THF, Diethylether, Ethylenglykoldimethylether) und deren Gemische.

Zur erfindungsgemäßen Herstellung einer Lösung von reinem TEDA, die z. B. als Katalysatorlösung bei der Polyurethanschaumherstellung verwendet werden kann, wird als Lösungsmittel für den TEDA-Quench bevorzugt ein Alkohol (z. B. Ethylenglykol, 1,4-Butandiol, Dipropylenglykol) eingesetzt. Die Farbzahl einer so erhaltenen 33 Gew.-%igen TEDA-Lösung in Dipropylenglykol beträgt kleiner 150 APHA, insbesondere kleiner 100 APHA.

Zur erfindungsgemäßen Herstellung von reinem (kristallinem) TEDA wird als Lösungsmittel für den TEDA-Quench bevorzugt ein aliphatischer Kohlenwasserstoff, insbesondere ein gesättigter aliphatischer Kohlenwasserstoff mit 5 bis 8 C-Atomen (wie z. B. Pentan, Hexan, Heptan) verwendet. Die Kristallisation des reinen TEDAs aus der erfindungsgemäß hergestellten TEDA-Lösung kann nach den dem Fachmann bekannten Verfahren erfolgen. Die durch eine nachfolgende mehrstufige, oder bevorzugt einstufige, Kristallisation erhaltenen TEDA-Kristalle sind hochrein (Reinheit von im allgemeinen mindestens 99,5 Gew.-%, insbesondere mindestens 99,9 Gew.-%) und die Farbzahl einer 33 Gew.-%igen Lösung in Dipropylenglykol beträgt kleiner 50, insbesondere kleiner 30, APHA.

Die erfindungsgemäße Einleitung des dampfförmigen TEDAs in das flüssige Lösungsmittel erfolgt in einem Quenchapparat, z. B. bevorzugt in einem Fallfilmkondensator (Dünnschicht-, Rieselfilm- oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDAs von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen um eine vollständige Benetzung der Innenwand des Quenchapparates zu erreichen.

Das Lösungsmittel kann im einmaligen Durchlauf oder als Kreislauflösung eingesetzt werden.

Die Menge des verwendeten Lösungsmittels ist nicht erfindungswesentlich und wird nach Zweckmäßigkeitsgesichtspunkten ausgewählt. Im allgemeinen wird so verfahren, dass, je nach Art des Lösungsmittels, Lösungen mit einem TEDA-Gehalt von ca. 1 bis 50 Gew.-%, bevorzugt 20 bis 40 Gew.-%, erhalten werden.

Im allgemeinen wird die Temperatur im TEDA-Quench durch Temperierung des eingesetzten Lösungsmittels und/oder des Quenchapparates auf 20 bis 100 °C, bevorzugt 30 bis 60 °C, eingestellt.

Der Absolutdruck im TEDA-Quench beträgt im allgemeinen 0,5 bis 1,5 bar.

Durch die partielle Verdampfung des eingesetzten Lösungsmittels infolge der Wärmezufuhr des dampfförmigen TEDAs ist der Gasraum im Quenchapparat mit Lösungsmitteldampf gesättigt. Dadurch wird die Desublimation des dampfförmigen TEDAs und die dadurch bedingten Verstopfungsprobleme durch Feststoffablagerung in den Austragsleitungen deutlich reduziert oder völlig verhindert.

Das im erfindungsgemäßen Verfahren eingesetzte und zu verdampfende TEDA kann nach den bekannten Verfahren, z. B. durch Umsetzung von Monoethanolamin, Diethanolamin, Triethanolamin, Ethylendiamin, Diethylentriamin, Triethylentetramin, Piperazin, N-(2-Hydroxyethyl)-piperazin, N,N'-Bis(2-Hydroxyethyl)-piperazin, N- (2-Aminoethyl)-piperazin, N,N'-Bis(2-Aminoethyl)-piperazin, Morpholin oder Mischungen hiervon an einem Katalysator [z. B. Metallpyrophosphate, Metallphosphate (wie Erdalkalimonohydrogenphosphat), Zeolithe, Zirkoniumphosphate, Al₂O₃, SiO₂, phosphorhaltiges TiO₂ oder ZrO₂] bei erhöhter Temperatur (im allgemeinen 250 bis 450 °C), erhalten werden. Üblicherweise beträgt hierbei der Druck 0,1 bis 50, insbesondere 0,1 bis 5, bar. Optional kann die Umsetzung in Gegenwart eines inerten polaren aprotischen Lösungsmittels (z. B. N-Alkylpyrrolidon (wie N-Methylpyrrolidon), Dioxan, THF, Dialkylformamid (wie Dimethylformamid), Dialkylacetamid (wie Dimethylacetamid)) und eines inerten Trägergases (z. B. N₂ oder Ar) durchgeführt werden.

Das so erhaltene TEDA kann durch Destillation und/oder Kristallisation gereinigt werden.

Derartige Verfahren sind z. B. beschrieben in DT-A-24 42 929, US-A-3,297,701, DE-A-36 34 258, DE-A-1 745 627, DE-A-37 18 395, EP-A-111 928, EP-A-382 055, EP-A-842 935, EP-A-842 936, EP-A-831 096, EP-A-952 152 und US-A-5,741,906.

Die Verdampfung des im erfindungsgemäßen Verfahren als Ausgangsprodukt verwendeten TEDAs, das die Qualitätsanforderungen bezüglich Farbe, Farbstabilität, Geruch und/oder Reinheit nicht erfüllt, kann nach den dem Fachmann geläufigen Verfahren und Bedingungen erfolgen, z. B. in einer Destillationsapparatur, wobei das TEDA oder ein Gemisch enthaltend das TEDA (rohes TEDA) vorgelegt wird. Bevorzugt wird das dampfförmige TEDA am Kopf oder in einem Seitenabzug einer Destillationskolonne erhalten. Das im erfindungsgemäßen Verfahren verwendete dampfförmige TEDA besitzt im allgemeinen eine Reinheit von größer 90 Gew.-%, bevorzugt größer 95 Gew.-%, insbesondere größer 97 Gew.-%.

Die Zeitdauer zwischen Anfall des im erfindungsgemäßen Verfahren verwendeten dampfförmigen TEDAs und TEDA-Quench beträgt vorteilhafterweise ≤ 10 Sekunden.

Gemäß einer bevorzugten Ausführungsform lässt sich das erfindungsgemäße Verfahren wie folgt ausführen:

Eine Mischung enthaltend das zu verdampfende TEDA, die z. B. in einem kontinuierlichen Verfahren durch Umsetzung von N-(2-Hydroxyethyl)-piperazin in einem Gasphasenreaktor bei 320 bis 420 °c und 0,5 bis 1,5 bar in Gegenwart eines polaren aprotischen Lösungsmittels (z. B.: N-Alkylpyrrolidon (wie N-Methylpyrrolidon), Dioxan, THF, Dialkylformamid (wie Dimethylformamid), Dialkylacetamid (wie Dimethylacetamid)), eines Trägergases (z. B. N₂ oder Ar) und eines Erdalkalimonohydrogenphosphats als Katalysator erhalten wurde, wird in eine Destillationsapparatur mit einer Destillationskolonne mit z. B. ca. 30 theoretischen Stufen geleitet. Hier werden Leichtsieder (wie z. B. Wasser, Piperazin, N-Ethylpiperazin) bei einer Kopftemperatur von 100 °C bis 120 °C und einem Druck von im allgemeinen 500 mbar bis 1,5 bar über Kopf abgetrennt.

Der Sumpfablauf wird in eine weitere Destillationskolonne mit z. B. ca. 25 theoretischen Stufen gepumpt. Bei einem Druck von im allgemeinen 500 mbar bis 1,5 bar wird in dieser Kolonne das gegebenenfalls bei der Synthese von TEDA verwendete Lösungsmittel aus dem Seitenabzug abgetrennt und optional wieder zurück zum Synthesereaktor gefahren und die Schwersieder werden über den Sumpfablauf ausgeschleust. Am Kopf der Kolonne wird TEDA mit einer Reinheit von größer 95 Gew.-%, insbesondere größer 97 Gew.-%, über einen Teilkondensator dampfförmig abgezogen und in einem Fallfilmkondensator in einem Lösungsmittel (z. B. Pentan, Dipropylenglykol) bei einer Temperatur von im allgemeinen 30 bis 100 °C, bevorzugt 30 bis 60 °C, direkt schockartig abgekühlt und gleichzeitig gelöst (TEDA-Quench).

### Beispiele

### Beispiel 1

Die Versuche wurden in einem mit elektrischen Heizbändern beheizten 4 1 (Katalysatorvolumen) Salzbadreaktor (Rohrbündel aus 7 Rohren, Innendurchmesser 21 mm, Länge 2 m) aus rostfreiem Stahl durchgeführt. Die Rohrleitungen für den Reaktorfeed, Reaktoraustrag und den Destillationsteil waren zum Teil als Doppelmantelrohre ausgeführt und ölbeheizt. Die Anlagenteile waren schutzbeheizt und wurden durch Einsatz verschiedener Heizkreisläufe an die jeweils erforderliche Temperatur individuell angepaßt.

Als Katalysator wurde CaHPO₄ in Form von Tabletten (Durchmesser ca. 3 mm, Höhe ca. 3 mm) verwendet (Katalysatorschüttung).

Der Katalysator wurde wie folgt hergestellt:

CaHPO₄ x 2 H₂O wurde 16 h bei ca. 250 °C in einem mit einer Entlüftungseinrichtung ausgestatteten Trockenschrank getempert. Anschließend wurde dem erhaltenen Pulver 2 Gew.-% Graphit zugemischt und die erhaltene Pulvermischung bei 270 bar mit Hilfe einer Strangpresse zu Tabletten verpresst.

Das Einsatzgut von 1600 g/h sowie 150 Nl/h (Nl = Normliter = auf Normalbedingungen umgerechnetes Volumen) Stickstoff wurden bei Normaldruck in den auf 380 °C beheizten Salzbadreaktor geleitet (Katalysatorbelastung: 0,4 kg Einsatzgut pro l Kat. (Schüttvolumen) und pro h)).

Das Einsatzgut hatte folgende Zusammensetzung (Angaben in Gew.-%):

| | |
|---|---|
| N-(2-Hydroxyethyl)-piperazin | 33 % |
| N,N'-Di-(2-Hydroxyethyl)-piperazin | 12 % |
| Piperazin | 15 % |
| N-Methylpyrrolidon | 19 % (als Lösungsmittel) |
| Wasser | 21 % |

Das dampfförmige Reaktionsprodukt wurde in einem Quench mit Kreislaufflüssigkeit, die aus zuvor erhaltenem flüssigem Reaktionsprodukt bestand (siehe unten), bei 60 °C kondensiert (= Reaktionsautrags-Quench).

Die Analyse des Kondensats ergab folgende Zusammensetzung (Angaben in Gew.-%):

| | |
|---|---|
| Piperazin | 7 % |
| Ethylpiperazin | 3 % |
| Triethylendiamin (TEDA) | 29 % |
| N-Methylpyrrolidon | 23 % |
| Wasser | 27 % |
| Rest | Schwersieder und andere Nebenprodukte |

Die nicht kondensierten Anteile wurden nach einem Gas/Flüssig-Abscheider über eine Kühlfalle und eine anschließende Waschflasche abgeleitet.

Ein Teil des flüssigen Reaktionsproduktes wurde gekühlt und als Flüssigkeitskreislauf (für den Reaktionsaustrags-Quench) verwendet, ein anderer Teil wurde kontinuierlich über eine Pumpe in eine Destillationskolonne (K 1) gepumpt. Die Glaskolonne mit einem Durchmesser von 50 mm war mit 60 Glockenböden ausgerüstet. Das Rücklaufverhältnis betrug etwa 1,4 : 1. Die Leichtsieder (Wasser, Piperazin, Ethylpiperazin) wurden bei Normaldruck und einer Kopftemperatur von 116 °C am Kopf der Kolonne flüssig abgezogen.

Die Analyse der Leichtsiederfraktion ergab folgende Zusammensetzung (Angaben in Gew.-%):

| | |
|---|---|
| Piperazin | 17 % |
| Ethylpiperazin | 9 % |
| Triethylendiamin (TEDA) | 2 % |
| Wasser | 63 % |
| Rest | Nebenprodukte |

Der Sumpfablauf der Destillationskolonne wurde bei 184 °C kontinuierlich in eine nachfolgende Destillationskolonne K 2 gepumpt. Die Glaskolonne K 2 mit einem Durchmesser von 50 mm war mit 50 Glockenböden ausgerüstet. Das Rücklaufverhältnis betrug etwa 10 : 1. Das Lösungsmittel N-Methylpyrrolidon wurde bei einer Temperatur von 208 °C aus einem Seitenabzug oberhalb des 1. Glokkenbodens abgezogen und wieder zum Reaktor geleitet, die Schwersieder wurden bei 225 °C über den Kolonnensumpf ausgeschleust. Am Kopf der Kolonne wurde TEDA dampfförmig abgezogen und bei ca. 30 °C im flüssigen Lösungsmittel Pentan (Mischung aus 80 Gew.-% n-Pentan und 20 Gew.-% iso-Pentan) schockartig abgekühlt und gleichzeitig gelöst (= TEDA-Quench). Für den TEDA-Quench wurde ein Fallfilmkondensator (Rieselfilm- oder Fallstromkondensator) eingesetzt, bei dem dampfförmiges TEDA von oben eingeleitet wurde. Die Pentanzufuhr erfolgte tangential am Kopf des Fallfilmkondensators. Die resultierende Lösung hatte folgende Zusammensetzung (Angaben in Gew.-%):

| | |
|---|---|
| Piperazin | 0,02 % |
| Ethylpiperazin | 0,02 % |
| Triethylendiamin (TEDA) | 7,5 % |
| N-Methylpyrrolidon | 0,01 % |
| Pentan | 92 % |

Nach der Abtrennung von Pentan durch Kühlungskristallisation bei 0 °C unter Stickstoff wurde TEDA in einer Reinheit von mindestens 99,5 Gew.-% erhalten.

Eine 33 Gew.-%ige Lösung des erhaltenen TEDAs in Dipropylenglykol (DPG) besaß eine APHA-Farbzahl von 28.

Das erhaltene TEDA wies keinen Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen auf.

### Beispiel 2

Bei Durchführung des Versuchs wie in Beispiel 1 beschrieben, jedoch unter Verwendung von Dipropylenglykol (DPG) anstelle von Pentan als Lösungsmittel für den TEDA-Quench und ohne anschließende Kristallisation des TEDAs aus dem Lösungsmittel wurde folgendes Ergebnis erhalten.

| Zusammensetzung der TEDA/DPG-Lösung (Angaben in Gew.-%): | |
|---|---|
| Piperazin | 0,03 % |
| Ethylpiperazin | 0,06 % |
| Triethylendiamin (TEDA) | 25 % |
| N-Methylpyrrolidon | 0,01 % |
| Dipropylenglykol | 73 % |

Diese TEDA/DPG-Lösung besaß eine APHA-Farbzahl von 140 und kann direkt als Katalysator bei der Herstellung von Polyurethanen eingesetzt werden.

Die erhaltene TEDA/DPG-Lösung wies keinen Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen auf.

### Vergleichsbeispiel

Bei Durchführung des Versuchs wie in Beispiel 1 beschrieben, wobei jedoch TEDA flüssig am Kopf der Glaskolonne K 2 abgezogen und kein TEDA-Quench durchgeführt wurde, wurde folgendes Ergebnis erhalten.

| Zusammensetzung des Destillats (Angaben in Gew.-%): | |
|---|---|
| Piperazin | 0,07 % |
| Ethylpiperazin | 0,06 % |
| Triethylendiamin (TEDA) | 98,8 % |
| N-Methylpyrrolidon | 0,01 % |

Die Kondensation von TEDA in einem Kondensator nach konventioneller Art und das Weiterleiten des flüssigen TEDAs bei den hierfür erforderlichen hohen Temperaturen (> 160 °C), z. B. in die Destillationsvorlage, führt zu erheblichen thermischen Belastungen des TEDAs und zur Bildung unerwünschter Zersetzungsprodukte.

Das so gewonnene TEDA wies bezüglich seiner Farbe und seines Geruches ungenügende Eigenschaften auf und war deshalb nicht marktfähig:
APHA-Farbzahl einer 33 Gew.-%igen Lösung in Dipropylenglykol (DPG): 1000,
Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen.

Eine Schichtkristallisation des erhaltenen TEDAs als weitere Reinigungsstufe konnte die TEDA-Qualität bezüglich des Geruchs nicht entscheidend verbessern:
APHA-Farbzahl einer 33 Gew.-%igen Lösung in Dipropylenglykol (DPG): 130,
Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen.

## Patentansprüche

1. Verfahren zur Herstellung einer Lösung von reinem Triethylendiamin (TEDA), **dadurch gekennzeichnet, dass** man TEDA verdampft und das dampfförmige TEDA in ein flüssiges Lösungsmittel einleitet.

2. Verfahren nach Anspruch 1, **dadurch gekennzeichnet, dass** man das dampfförmige TEDA am Kopf oder in einem Seitenabzug einer Destillationskolonne erhält.

3. Verfahren zur Herstellung von reinem Triethylendiamin (TEDA), **dadurch gekennzeichnet, dass** man eine Lösung von reinem TEDA gemäß den Ansprüchen 1 oder 2 herstellt und anschließend das TEDA aus dieser Lösung auskristallisiert.

4. Verfahren nach den Ansprüchen 1 bis 3, **dadurch gekennzeichnet, dass** das flüssige Lösungsmittel aus der Gruppe cyclische oder acyclische Kohlenwasserstoffe, chlorierte aliphatische Kohlenwasserstoffe, aromatische Kohlenwasserstoffe, Alkohole, Ketone, aliphatische Carbonsäureester, aliphatische Nitrile und Ether ausgewählt wird.

5. Verfahren nach den Ansprüchen 1 bis 3, **dadurch gekennzeichnet, dass** man als flüssiges Lösungsmittel Pentan oder Dipropylenglykol einsetzt.

6. Verfahren nach den Ansprüchen 1 bis 5, **dadurch gekennzeichnet, dass** das dampfförmige TEDA zur Einleitung in das flüssige Lösungsmittel eine Reinheit von größer 95 Gew.-% besitzt.

7. Verfahren nach den Ansprüchen 1 bis 6, **dadurch gekennzeichnet, dass** das zu verdampfende TEDA durch Umsetzung von Monoethanolamin, Diethanolamin, Triethanolamin, Ethylendiamin, Diethylentriamin, Triethylentetramin, Piperazin, N-(2-Hydroxyethyl)-piperazin, N,N'-Bis(2-Hydroxyethyl)-piperazin, N-(2-Aminoethyl)-piperazin, N,N'-Bis(2-Aminoethyl)-piperazin, Morpholin oder Mischungen hiervon an einem Katalysator bei erhöhter Temperatur erhalten wurde.

8. Verfahren nach Anspruch 7, **dadurch gekennzeichnet, dass** es sich bei dem Katalysator um ein Metallphosphat oder einen Zeolith handelte.

9. Verfahren nach den Ansprüchen 7 und 8, **dadurch gekennzeichnet, dass** man die Umsetzung bei Temperaturen von 250 bis 450 °C in der Gasphase durchführte.

## Claims

1. A process for preparing a solution of pure triethylenediamine (TEDA), which comprises vaporizing TEDA and passing the gaseous TEDA into a liquid solvent.

2. A process as claimed in claim 1, wherein the gaseous TEDA is obtained at the top or at a side offtake of a distillation column.

3. A process for preparing pure triethylenediamine (TEDA), which comprises preparing a solution of pure TEDA as claimed in claim 1 or 2 and subsequently crystallizing the TEDA from this solution.

4. A process as claimed in any of claims 1 to 3, wherein the liquid solvent is selected from the group consisting of cyclic and acyclic hydrocarbons, chlorinated aliphatic hydrocarbons, aromatic hydrocarbons, alcohols, ketones, aliphatic carboxylates, aliphatic nitriles and ethers.

5. A process as claimed in any of claims 1 to 3, wherein the liquid solvent used is pentane or dipropylene glycol.

6. A process as claimed in any of claims 1 to 5, wherein the gaseous TEDA which is to be passed into the liquid solvent has a purity of greater than 95% by weight.

7. A process as claimed in any of claims 1 to 6, wherein the TEDA to be vaporized has been obtained by reaction of monoethanolamine, diethanolamine, triethanolamine, ethylenediamine, diethylenetriamine, triethylenetetramine, piperazine, N-(2-hydroxyethyl)piperazine, N,N'-bis(2-hydroxyethyl)piperazine, N-(2-aminoethyl)piperazine, N,N'-bis(2-aminoethyl)piperazine, morpholine or a mixture thereof over a catalyst at elevated temperature.

8. A process as claimed in claim 7, wherein the catalyst is a metal phosphate or a zeolite.

9. A process as claimed in claim 7 or 8, wherein the reaction is carried out in the gas phase at from 250 to 450°C.

## Revendications

1. Procédé de préparation d'une solution de triéthylènediamine (TEDA) pure, **caractérisé en ce qu'**on évapore du TEDA, et on introduit le TEDA sous forme vapeur dans un solvant liquide.

2. Procédé selon la revendication 1, **caractérisé en ce qu'**on obtient le TEDA sous forme vapeur en tête ou dans un soutirage latéral d'une colonne de distillation.

3. Procédé de préparation de triéthylènediamine (TEDA) pure, **caractérisé en ce qu'**on prépare une solution de TEDA pur selon les revendications 1 ou 2, puis on sépare le TEDA de cette solution par cristallisation.

4. Procédé selon les revendications 1 à 3, **caractérisé en ce que** le solvant liquide est choisi dans l'ensemble comprenant les hydrocarbures cycliques ou acycliques, les hydrocarbures aliphatiques chlorés, les hydrocarbures aromatiques, les alcools, les cétones, les carboxylates aliphatiques, les nitriles aliphatiques et les éthers.

5. Procédé selon les revendications 1 à 3, **caractérisé en ce qu'**on utilise en tant que solvant liquide le pentane ou le dipropylèneglycol.

6. Procédé selon les revendications 1 à 5, **caractérisé en ce que** le TEDA sous forme vapeur présente, pour introduction dans le solvant liquide, une pureté supérieure à 95 % en poids.

7. Procédé selon les revendications 1 à 6, **caractérisé en ce que** le TEDA à évaporer est obtenu par réaction sur un catalyseur à haute température de monoéthanolamine, de diéthanolamine, de triéthanolamine, d'éthylènediamine, de diéthylènetriamine, de triéthylènetétramine, de pipérazine, de N-(2-hydroxyéthyl)-pipérazine, de N,N'-bis(2-hydroxyéthyl)-pipérazine, de N-(2-aminoéthyl)-pipérazine, de N,N'-bis(2-aminoéthyl)-pipérazine, de morpholine ou de leurs mélanges.

8. Procédé selon la revendication 7, **caractérisé en ce que**, pour ce qui concerne le catalyseur, il s'agit d'un phosphate métallique ou d'une zéolite.

9. Procédé selon les revendications 7 et 8, **caractérisé en ce qu'**on met en oeuvre la réaction à des températures de 250 à 450°C en phase gazeuse.
